# EUROPEAN PATENT SPECIFICATION

(11) **EP 4 241 651 B1**
(45) Date of publication and mention of the grant of the patent: **02.12.2026**
(21) Application number: 21888921.0
(22) Date of filing: 13.09.2021
(51) Int. Cl.: A61B 3/024, A61B 3/00, A61B 3/14, A61B 3/032, A61B 3/113, A61B 5/00, A61B 5/16, G02B 27/01

(54) **CHARACTERISTIC TESTING SYSTEM**
EIGENSCHAFTSINSPEKTIONSSYSTEM
SYSTÈME D'INSPECTION DE CARACTÉRISTIQUES

(30) Priority: 05.11.2020 JP 2020184793
(43) Date of publication of application: 13.09.2023
(73) Proprietor: FINDEX Inc., Chiyoda-ku, Tokyo 100-0004 (JP)
(72) Inventor: AIBARA, Teruo, Matsuyama-shi, Ehime 790-0003 (JP)
(74) Representative: Hasegawa, Kan
(86) International application number: PCT/JP2021/033444
(87) International publication number: WO 2022/097368

(56) References cited:
- WO-A1-2008/139137
- WO-A1-2020/137544
- WO-A1-2020/179351
- JP-A- 2011 161 122
- JP-A- 2015 181 868
- JP-A- 2020 141 848
- US-A1- 2009 009 714

## Description

### TECHNICAL FIELD

The present invention relates to a characteristic testing system that carries out testing of user characteristics based on eye movement.

### BACKGROUND ART

Apparatuses that carry out testing of user characteristics based on movements of the eye of the user have been provided conventionally; for example, a visual field testing apparatus is disclosed at JP2011-161122A, in which the visual field of a user is measured by causing the line of sight which is the direction in which the user is looking to be automatically detected by a line of sight detection apparatus, and automatically determining whether or not the user sees a target.

Furthermore, disclosed at JP2017-176302A, is an apparatus that uses eye movement to detect user dementia or detect reduced cognitive function or the like due to depressed mental state.

WO 2020/179351 A1 discloses a target visual confirmation determination system comprising a visual confirmation determination unit determining that a user has visually confirmed a target in the case where a target approach determination unit determines that a line of sight of the user has approached the target, and a stare determination unit determines that the line of sight remained in the vicinity of the predetermined coordinate position.

WO 2020/137544 A1 discloses a visual field test device in which the display position of a reference test optotype and the display position of a peripheral test optotype are associated with each other and stored as a single set of information, comprises a test optotype display function including a display position adjustment function which is for adjusting the display position of the reference test optotype and the display position of the peripheral test optotype on the basis of the relation between the display unit and the display position of the peripheral test optotype.

### SUMMARY OF INVENTION

### PROBLEM TO BE SOLVED BY INVENTION

However, apparatuses that use targets to carry out testing of user characteristics have been such that determination has been carried out as to whether or not all of the targets which are sequentially displayed have actually been seen by the user, as a result of which much time has been required for testing of user characteristics, and the burden placed on the user in connection with testing has been large.

The present invention was conceived in light of such problems, it being an object thereof to provide a characteristic testing system capable of efficiently testing user characteristics based on eye movement.

### MEANS FOR SOLVING PROBLEM

The problem is solved by the teachings of the independent claim. Further embodiments are defined in the dependent claims. The invention is defined by the appended claims.

In the context of a characteristic testing system that causes targets to be displayed at a display and that carries out testing of a characteristic of a user based on movement of a line of sight of the user, a characteristic testing system associated with the present disclosure is characterized in that it comprises a line of sight detection unit that detects the line of sight of the user and that outputs line of sight information pertaining to a direction of the line of sight; a target display unit that causes the targets to be displayed at the display; a visual recognition determination unit that carries out determination as to whether or not the user has visually recognized the targets based on the line of sight information and position information of the targets; and a characteristic testing unit that controls the target display unit and that carries out testing with respect to the characteristic of the user based on movement of the line of sight when the targets are displayed; wherein the characteristic testing unit comprises a multiple target testing unit that carries out testing with respect to the characteristic of the user when a plurality of the targets are simultaneously displayed at the display.

### BENEFIT OF INVENTION

The present invention makes it possible to efficiently carry out testing of user characteristics based on movement of the line of sight when a plurality of targets are displayed.

### BRIEF DESCRIPTION OF DRAWINGS

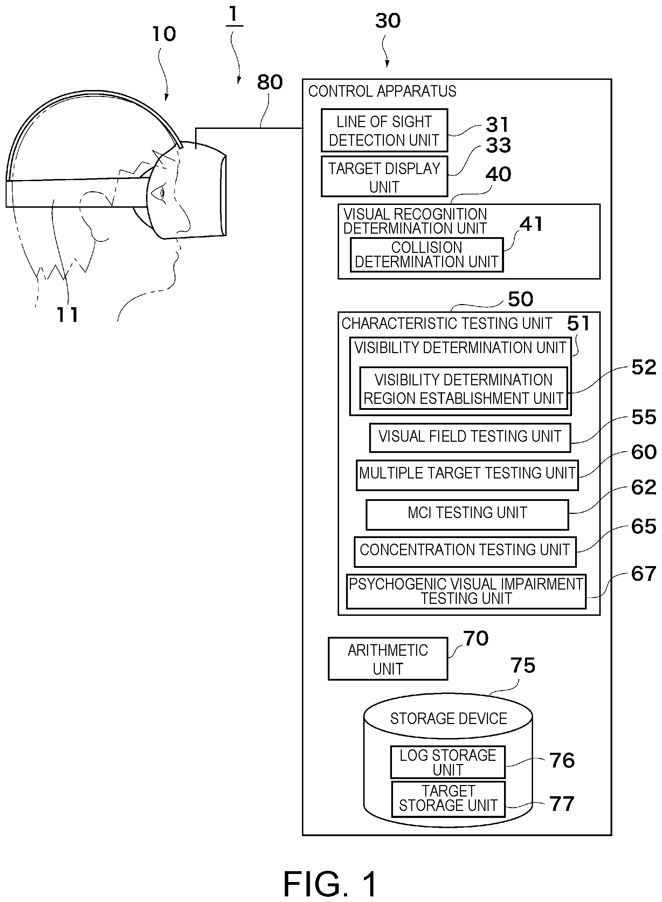

[FIG. 1] FIG. 1 is a schematic diagram showing in simplified fashion the constitution of a characteristic testing system associated with an embodiment of the present invention.

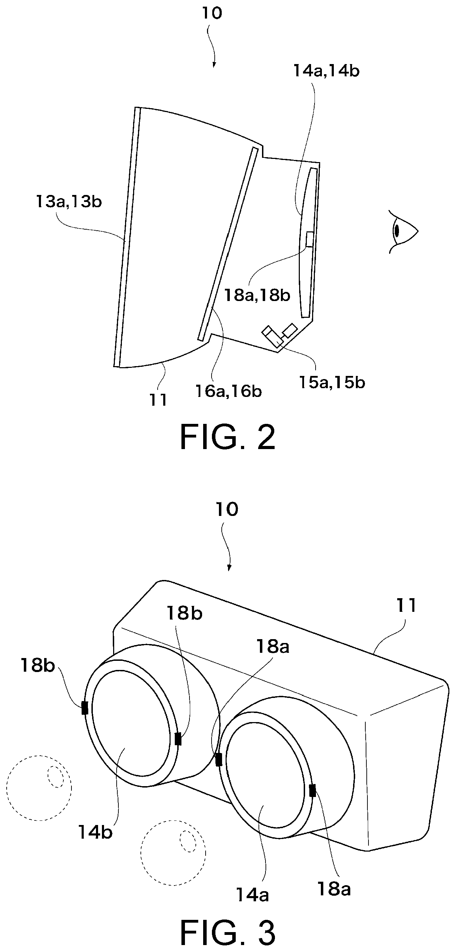

[FIG. 2] FIG. 2 is a sectional view of the principal components in, and showing in simplified fashion the constitution of, an HMD associated with an embodiment of the present invention.
[FIG. 3] FIG. 3 is a perspective view of the principal components in, and showing in simplified fashion the constitution of, an HMD associated with an embodiment of the present invention.
[FIG. 4] FIG. 4 is a drawing showing locations at which targets from a target display unit associated with an embodiment of the present invention might be displayed.
[FIG. 5] FIG. 5 is a flowchart showing flow of determination processing at a visibility determination unit associated with an embodiment of the present invention.
[FIG. 6] FIG. 6 is a drawing for describing a visibility determination region associated with an embodiment of the present invention.
[FIG. 7] FIG. 7 is a flowchart showing flow of processing during response testing carried out by a multiple target testing unit associated with an embodiment of the present invention.
[FIG. 8] FIG. 8 is a drawing showing locations at which multiple targets might be displayed during multiple target display response testing associated with an embodiment of the present invention.
[FIG. 9] FIG. 9 is a drawing showing locations at which multiple targets might be displayed during psychogenic visual impairment testing associated with an embodiment of the present invention.

### EMBODIMENTS FOR CARRYING OUT INVENTION

Characteristic testing system 1, which is an embodiment of the present invention, will be described in detail hereinbelow with reference to the drawings. Characteristic testing system 1 automatically detects a line of sight which is a direction in which the eye(s) of a user are looking, and carries out testing of user characteristics based on movement of the line of sight at a time when target(s) are displayed. The present embodiment is characterized in that testing of user characteristics is carried out as a plurality of targets are simultaneously displayed.

Characteristic testing system 1 comprises head mounted display (HMD) 10, control apparatus 30, and communication cable 80 which connects HMD 10 and control apparatus 30. HMD 10 comprises case 11 which includes a belt for mounting thereof on the head of a user who is a test subject, display 13, convex lens 14, camera 15, hot mirror 16, and near-infrared emitter 18, and is equipped with line of sight detection functionality for cooperating with line of sight detection unit 31, described below, to automatically detect a line of sight which is a direction in which the user on whom HMD 10 is mounted is looking with his or her eye(s).

Display 13 is a liquid crystal display in which display 13a for the right eye of the user and display 13b for the left eye thereof are installed so as to respectively face the fronts of his or her left and right eyes. Respectively installed to the right and left between display 13 and the eyes of the user are convex lens 14a for the right eye and convex lens 14b for the left eye. An image displayed at display 13 will appear at the eye(s) of the user by way of convex lens 14.

Camera 15 is a near-infrared camera for capturing images of the eyes of the user, video capture of the left and right eyes of the user being carried out thereby through use of near-infrared light which is nonvisible light. Camera 15a for the right eye and camera 15b for the left eye are likewise installed at camera 15.

Respectively installed to the right and left between display 13 and convex lens 14 are hot mirrors 16a, 16b having multilayer films which reflect near-infrared light and allow visible light to pass therethrough. Visible light of an image irradiated from display 13 passes through hot mirror 16, and near-infrared light which is nonvisible light that is irradiated from emitter 18 is reflected by hot mirror 16.

Emitter 18 is an LED (IR-LED) that causes near-infrared light to be irradiated as illumination for capturing images of the eyes of the user. Emitters 18a, 18b are respectively installed to the left and right so as to face the left and right eyes of the user at the periphery of convex lens 14.

Camera 15 is installed at the side toward the eyes, which is the side opposite display 13, from hot mirror 16. Near-infrared light which directly irradiates the eyes of the user from emitter 18 is reflected by the eyes of the user and thereafter passes through convex lens 14 and is reflected by hot mirror 16 to arrive at camera 15 where an image is captured therefrom.

Control apparatus 30 comprises central processing unit(s) (CPU) or other such arithmetic unit(s) 70 for carrying out various types of operations, as well as hard disc drive(s) (HDD) for storing various types of information, and/or random access memory or memories (RAM) capable of being used as a work area during arithmetic processing, or other such storage device(s) 75.

Storage device 75 comprises line of sight log storage unit 76 which records line of sight information that is output by line of sight detection unit 31, described below; and target storage unit 77 which records target information to be displayed at display 13.

Furthermore, control apparatus 30 comprises-in terms of functionalities-line of sight detection unit 31, target display unit 33, visual recognition determination unit 40, and visual field testing unit 50, these functionalities being implemented by causing prescribed program(s) stored at storage device 75 to be executed by arithmetic unit 70.

Line of sight detection unit 31 detects the direction in which the user is looking, i.e., the line of sight, based on captured image(s) of the eye(s) of the user which is the output of camera 15. After detecting the line of sight, line of sight detection unit 31 outputs two angles θ, ϕ which are polar coordinates (spherical coordinates) as line of sight information (line of sight angles) that indicate the direction of the line of sight. In accordance with the present embodiment, taking a location at front and center to be the origin, the angle θ indicates the angle (°) in the horizontal direction (lateral direction) from a line of sight directed toward the location at front and center (the origin), and the angle ϕ likewise indicates the angle (°) in a perpendicular direction (vertical direction) from the location at front and center.

Establishment of the foregoing polar coordinate system may be carried out during the calibration which takes place prior to start of visual field measurement. More specifically, the user might be made to stare at a previously designated target and the image thereof might be made to correspond to the polar coordinate system origin (center of eyeball), following which an image at which the user is made to stare at a point representing a different designated set of polar coordinates might be stored. By determining the correlation between the locations of the centers of the pupils captured in the respective images and the respective polar coordinates at which the user was made to stare, it is possible to determine the line of sight angles (θ, ϕ) within the polar coordinate system from the images.

This line of sight detection that is carried out by line of sight detection unit 31 is carried out independently for the right eye and the left eye, line of sight information being recorded as a function of time at line of sight log storage unit 76 at intervals of approximately 15 ms. Included within the line of sight information which is recorded at line of sight log storage unit 76 are the aforementioned line of sight angles (θ, ϕ).

Target display unit 33 causes targets of prescribed size(s) to be displayed at prescribed locations(s) at display 13 based on target information recorded at target storage unit 77. At the target information stored at target storage unit 77, note that sets of the aforesaid two polar coordinate system angles θ, ϕ may be used as location information indicating display locations of respective targets, it being possible to define a target angle (θ, ϕ) indicating the display location of a target based on the horizontal angle θ and the vertical angle ϕ.

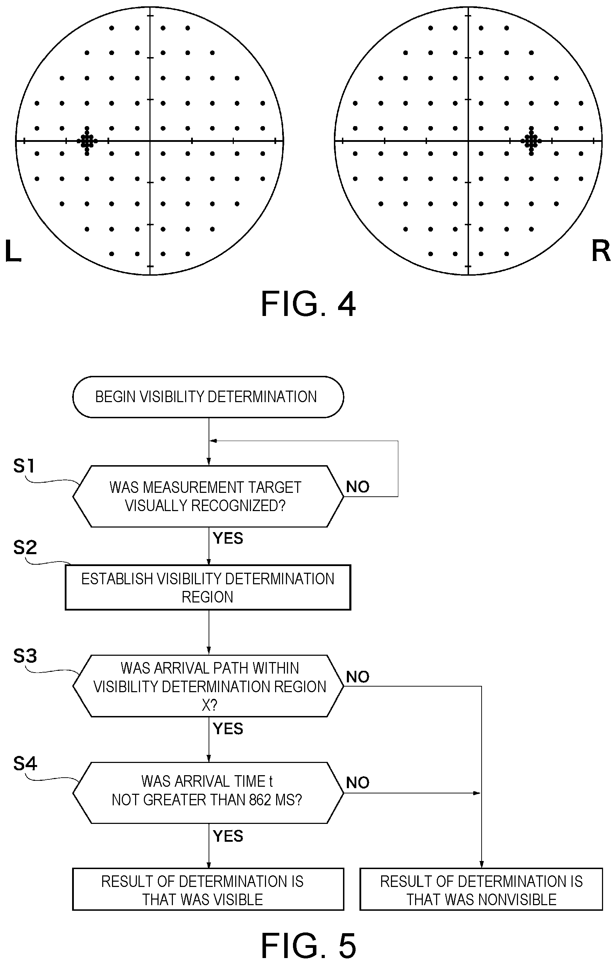

FIG. 4 shows display locations of targets displayed at display 13, horizontal angles θ being plotted on the horizontal axis, and vertical angles ϕ being plotted on the vertical axis. Tick marks are shown on the respective axes for 10°, 20°, and 30°. When the user visually recognizes a target that is displayed at a location having a target angle (θ, ϕ), this constitutes a visual field angle (θ, ϕ). In accordance with the present embodiment, targets are displayed within a visual field angle of 30° in both the horizontal direction and the vertical direction from such a location at front and center.

Note that when respective targets are displayed at display 13, target display unit 33 causes a target to be displayed at a location in the plane of display 13 that is the extension of an imaginary line segment drawn so as to connect the origin and the target which is displayed at a target angle with polar coordinates centered on the eye of the user so as to cause the angle of the line of sight that exists at a time when the line of sight of the user arrives at the target and is visually recognized to be the same as the aforesaid target angle of said target.

Visual recognition determination unit 40 which comprises collision determination unit 41 carries out determination as to whether or not the line of sight of the user has arrived at the target, i.e., whether or not the target has actually been visually recognized by the user, based on the line of sight detected by line of sight detection unit 31 and the coordinates of the target displayed at display 13.

Collision determination unit 41 carries out determination as to whether or not the target has been visually recognized by the user based on whether or not an imaginary line that is an extension of a line of sight direction detected by line of sight detection unit 31 within a prescribed coordinate system would physically collide with the target in question. More specifically, a determination will be made by collision determination unit 41 that there has been a collision and that visual recognition has taken place if upon comparison of the line of sight angle detected by line of sight detection unit 31 and the target angle indicating the location of the target in question, the angular difference therebetween is within 2°.

Note that it is also possible for visual recognition determination unit 40 to cause visual recognition determination to be carried out using logical determinative techniques that make use of logged line of sight information. For example, a constitution may be adopted in which visual recognition determination unit 40 causes logical determination of visual recognition to be carried out when the line of sight approaches the target in question and continues to be located (staring takes place) near said target.

Characteristic testing unit 50, which has functionality for testing various characteristics of a user who uses characteristic testing system 1, comprises visibility determination unit 51, visual field testing unit 55, multiple target testing unit 60, mild cognitive impairment (MCI) testing unit 62, concentration testing unit 65, and psychogenic visual impairment testing unit 67.

Visibility determination unit 51, which comprises visibility determination region establishment unit 52, carries out determination as to whether or not a target at a position other than a position at display 13 which is in the line of sight of the user has been seen by (has caught the eye of) the user, i.e., whether or not it is visible.

After the target has been displayed at display 13, visibility determination unit 51 carries out visibility determination based on the arrival path which is the path taken by the line of sight, and the arrival time which is the time taken by the line of sight, in going from the position that the line of sight was in at the time that display occurred until it arrives at said target. Determination as to whether or not the line of sight of the user arrived at the target is carried out by visual recognition determination unit 40.

Regarding the arrival path, visibility determination unit 51 carries out determination as to whether or not the path (arrival path) taken by the line of sight in going from the position that the line of sight was in at the time that the target was displayed until it arrived at the target was within a prescribed visibility determination region X.

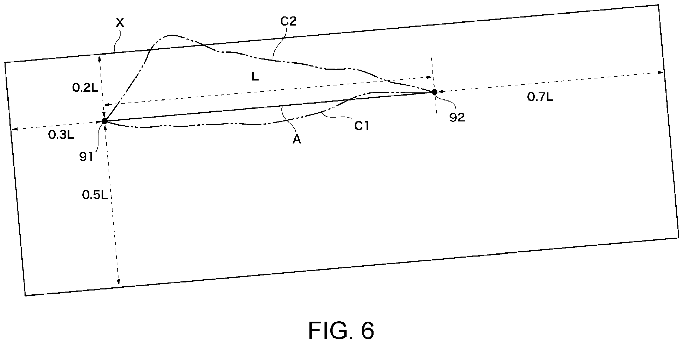

Visibility determination region establishment unit 52 establishes this visibility determination region X (see FIG. 6). In accordance with the present embodiment, taking a line segment which connects two points-these being the initial position 91 of the line of sight when a target is displayed and the position at which said target 92 is displayed-to be line segment A, visibility determination region establishment unit 52 causes visibility determination region X to be established as a rectangle which contains line segment A at the interior thereof and which is such that the long sides thereof are parallel to line segment A.

Describing this in further detail, taking the length of line segment A to be L, visibility determination region X might be established such that the distance between the initial position 91 of the line of sight and that short side of visibility determination region X which is toward line of sight 91 is 0.3 L, the distance between the position of target 92 and that short side thereof which is toward target 92 is 0.7 L, the distance between line segment A and that long side thereof which is to the right of the direction in which the line of sight proceeds in going from initial position 91 to target 92 is 0.5 L, and the distance between line segment A and that long side thereof which is to the left of the direction in which the line of sight proceeds in going from initial position 91 to target 92 is 0.2 L. That is, a rectangular visibility determination region X might be established such that the lengths of the long sides thereof are 2 L, and the lengths of the short sides thereof are 0.7 L.

Note that the aforementioned visibility determination region X is for the right eye; the extent thereof from the line of sight to the target would be flipped horizontally if for the left eye. That is, visibility determination region X for the left eye would span a greater distance to the left of the direction in which the line of sight proceeds than it spans to the right of the direction in which the line of sight proceeds.

Regarding the arrival time, visibility determination unit 51 carries out determination as to whether or not the arrival time t taken in going from the initial position 91 that the line of sight was in at the time that the target was displayed until it arrived at target 92 was not greater than 862 ms.

The flow of processing by which visibility determination is carried out by visibility determination unit 51 will next be described with reference to FIG. 5. At S1, visibility determination unit 51 ascertains whether visual recognition determination unit 40 has determined whether or not target 92 was visually recognized by the user, processing proceeding to S2 if it was visually recognized.

At S2, visibility determination region establishment unit 52 establishes visibility determination region X; at S3, visibility determination unit 51 ascertains whether or not the arrival path C was within visibility determination region X. If this was within visibility determination region X (C1 at FIG. 6), processing proceeds to S4; if arrival path C passed outside of visibility determination region X (C2 at FIG. 6), determination is made to the effect that said target 92 was nonvisible, and determination processing terminates.

Upon processing having proceeded to S4, visibility determination unit 51 ascertains whether or not the arrival time t was not greater than 862 ms. If this was not greater than 862 ms, determination is made to the effect that said target 92 was visible; if the arrival time was greater than 862 ms, determination is made to the effect that said target 92 was nonvisible, and determination processing terminates.

During visibility determination, the reason that whether arrival path C was within visibility determination region X is ascertained is because if this deviates greatly from the shortest path (line segment A) that could be taken by the line of sight in going from initial position 91 to the target 92, it is thought that this would likely have been due to the fact that presence of a scotoma at the time that the target was displayed prevented the user from seeing the target, and that it was only after the line of sight had been made to wander about that the target 92 happened to have been moved out of the scotoma and that this is what made it possible for the target 92 to be seen somewhere along the way.

Furthermore, during visibility determination, the reason that whether the arrival time t was not greater than a prescribed time is ascertained is because if the arrival time which is the time taken by the line of sight in going from initial position 91 to the target 92 is longer than a prescribed time, it is likewise thought that this would likely have been due to the fact that the user was unable to see the target 92 at the time that the target was displayed.

As described above, based on the arrival path which is the path taken by the point of sight, and the arrival time which is the time taken by the point of sight, in going from the initial position 91 of the point of sight at the time that the target was displayed until it arrived at the target 92, visibility determination unit 51 carries out determination to retroactively determine whether or not, at the time that a target was displayed, said target 92 was visible to the user.

Note that the shape and size of visibility determination region X, and the determination threshold (862 ms) for arrival time t, should be established empirically based on historical measurement data, and may be varied as appropriate. For example, instead of being rectangular, the shape of visibility determination region X might be elliptical or in the shape of a polygon other than a rectangle, and the size thereof may be varied as appropriate.

Note with respect to empiricism, however, that it is preferred that visibility determination region X span a greater distance to the right of the direction in which the arrival path proceeds if for the right eye, and that it span a greater distance to the left of the direction in which the arrival path proceeds if for the left eye; and also that it span a greater distance in the direction in which the line of sight proceeds in going from the initial position 91 to the target 92 than it does in the reverse direction.

Furthermore, because it is necessary that the outer edge of visibility determination region X not be too far away from the shortest path (line segment A) that could be taken by the line of sight in going from initial position 91 to target 92, it is preferred that the size of visibility determination region X be such as to cause the outer edge of visibility determination region X to be located within distance L from line segment A.

Furthermore, whereas in accordance with the present embodiment visibility determination is carried out based on both the arrival path and the arrival time, visibility determination may be carried out based on one of either the arrival path or the arrival time.

Visual field testing unit 55 controls target display unit 33, visual recognition determination unit 40, and visibility determination unit 51, and causes visual field testing of a user to be carried out by sequentially causing determination with respect to visibility of respective measurement targets to be carried out by visibility determination unit 51 while causing measurement targets for testing of visual field to be sequentially displayed at prescribed locations by target display unit 33.

As methods for causing targets to be displayed when visual field testing is carried out, target display unit 33 is equipped with a mode employing two types of targets-these being gazing targets for causing the line of sight to return to the vicinity of a location at front and center with respect to which gazing (ocular fixation) is made to take place, and measurement targets for measuring visual field-and a mode that does not employ gazing targets but only employs measurement targets. In accordance with the present embodiment, visual field testing is fundamentally carried out in the mode that employs only measurement targets. Visual field testing may be carried out in accordance with Humphrey visual field testing (30-2 center) or the like.

Response testing in which multiple targets are displayed by multiple target testing unit 60 will now be described. During processing by visibility determination unit 51, visual field testing unit 55, MCI testing unit 62, concentration testing unit 65, and psychogenic visual impairment testing unit 67, results of multiple target display response testing by this multiple target testing unit 60 are employed for the purpose of improving the efficiency of the respective tests.

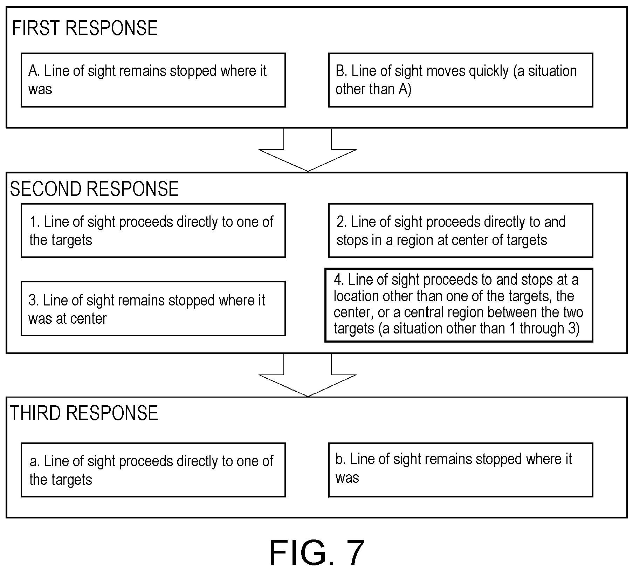

Multiple target testing unit 60 is configured to examine a response of movement of the user's line of sight when two targets are simultaneously displayed at display 13, and examine, in a time-series manner, a first response, second response, and third response as shown in FIG. 7.

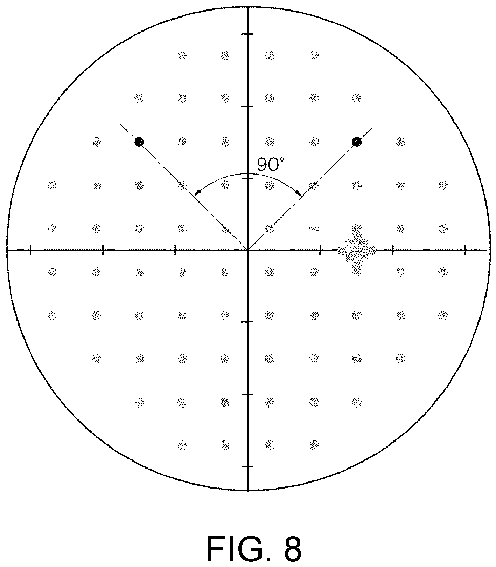

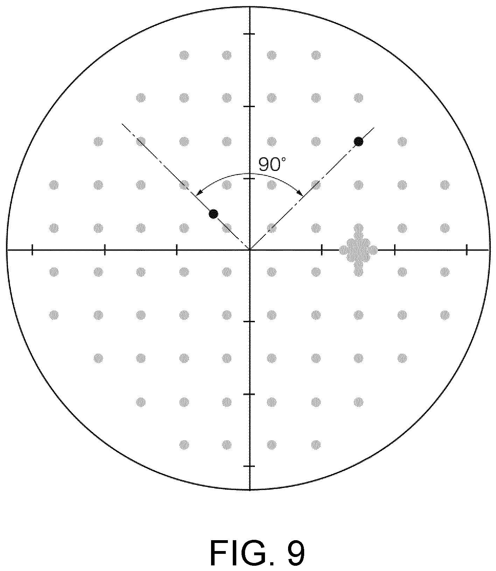

Multiple target testing unit 60 first causes two targets to be simultaneously displayed. FIG. 8 is a drawing showing locations at which two targets might be displayed during multiple target display response testing, display locations of targets being shown as target angles in a polar coordinate system in similar fashion as described above with reference to FIG. 4. At same drawing, dark black points indicate targets, and light gray points indicate locations at which targets are capable of being displayed, and the same thing is true with respect to FIG. 9.

The two targets are equidistant from the location of the current line of sight, being displayed at locations 90° apart in a clockwise direction centered on the location of the line of sight at FIG. 8. Whereas the situation that exists when the line of sight is located at the central origin is shown by way of example at same drawing, even where the line of sight is at a location other than the origin, multiple target testing unit 60 will cause two targets to be displayed in similar fashion at locations that are equidistant and 90° apart in a clockwise direction centered on the location of the current line of sight.

The display locations of the two targets are equidistant from the location of the current line of sight.

After causing two targets to be simultaneously displayed, multiple target testing unit 60 first carries out determination in connection with the first response. During the first response, multiple target testing unit 60 carries out determination as to whether or not, immediately following display of targets, the line of sight remained stopped where it was at its initial position. This is determined to be
"A" if the line of sight remained stopped where it was; this is determined to be
"B" if it quickly began to move therefrom.

Here, determination with respect to staring, in which determination is made as to whether or not the line of sight remains stopped, i.e., determination as to whether or not the user continues to look in the vicinity of a prescribed coordinate location, might be carried out by determining that the user is staring at said coordinate location if, for example, the respective components of the most-recent prescribed number of (e.g., 15) sets of line of sight information recorded at line of sight log storage unit 76 are not greater than ±2° from the target angle of said coordinate location.

The method used to carry out determination with respect to staring may of course be varied as appropriate, it being possible, for example, to respectively calculate standard deviation(s) for the most-recent prescribed number of sets of line of sight information, and to determine that staring is taking place when value(s) of standard deviation(s) for all components are not greater than prescribed threshold value(s).

Following the first determination, a second determination is carried out. During the second determination, when the line of sight proceeds directly to and then stops at either of the targets, this is determined to be "1"; when the line of sight proceeds directly to and stops in the vicinity of a central location between the two targets, this is determined to be "2"; when the line of sight does not move but remains stopped where it was at its initial position, this is determined to be "3"; and when something other than 1 through 3 occurs, this is determined to be "4".

A "4" at the second response determination means that the line of sight stops at a location other than the two targets, midway between the two targets, or the initial position. Note that the line of sight ordinarily does not keep moving forever but will stop somewhere after having had a chance to move. Furthermore, in the case of "1" or "2" at the second determination, determination as to whether or not the line of sight proceeds directly thereto may be carried out through visibility determination using visibility determination region X by the foregoing visibility determination unit 51.

Following the second determination, a third determination is carried out. During the third determination, following the second determination, determination is carried out as to whether or not the line of sight proceeds directly toward either target, this being determined to be "a" if it proceeds to either line of sight, and this being determined to be "b" if it remains stopped where it was. Here as well, determination as to whether or not the line of sight proceeds directly thereto may be carried out through visibility determination using visibility determination region X by visibility determination unit 51.

At the first determination through the third determination described above, each determination is carried out within 250 ms, the determination in each case being that the target remains stopped (the determination being that staring is taking place) if it remains stopped where it was despite passage of 250 ms following the start of the determination.

Description having been given with respect to the content of determinations that may be carried out by multiple target testing unit 60, description will now be given with respect to what respective combinations of results (the first determination, the second determination, and the third determination) obtained at the first through third determination mean in terms of movement of the line of sight.

A, 1, a: There was a delay in initial movement, and both targets were visually recognized.
A, 1, b: There was a delay in initial movement, and one of the targets was seen
A, 2, a: There was a delay in initial movement, there was anxiety regarding which target to look at, and one of the two was seen.
A, 2, b: There was a delay in initial movement, there was anxiety regarding which target to look at, but neither was seen.
A, 3, a: There was considerable delay in initial movement, and one of the targets was seen.
A, 3, b: Search was not attempted.
A, 4, a: There was a delay in initial movement, search was attempted, and one of the targets was seen.
A, 4, b: There was a delay in initial movement, search was initiated, but the attempt to search was abandoned.

B, 1, a: Both targets were quickly seen.
B, 1, b: One target was quickly seen.
B, 2, a: There was a quick response, there was anxiety regarding which target to look at, and one of the two was seen.
B, 2, b: There was a quick response, there was anxiety regarding which target to look at, but neither was seen.
B, 3, a: (Nonexistent combination)
B, 3, b: (Nonexistent combination)
B, 4, a: There was a quick response, search was attempted, and one of the targets was seen.
B, 4, b: There was a quick response, search was initiated, but the attempt to search was abandoned.

Various characteristics tests which might be performed by characteristic testing unit 50 using results of testing from multiple target testing unit 60 will be described next. Visibility determination carried out by visibility determination unit 51 will first be described. Based on the first determination and the second determination, visibility determination unit 51 carries out determination as to whether or not both of the two targets displayed at locations other than the location of the line of sight are visible.

When the result of determination at multiple target testing unit 60 is response (A, 2) or response (B, 2), visibility determination unit 51 determines that both of the two targets were visible at the time that the targets were displayed.

This indicates that at the time of termination of the second response the line of sight of the user had proceeded to a location midway between the two targets and had stopped there, it being thought that this was due to the fact that at the time that the targets were displayed both of the two targets were seen by the user, the fact that the line of sight was stopped midway between the two targets being thought to be a result of the user's having been unable to decide which to move the line of sight to.

As described above, determination as to whether or not a target was visible at the time that the target was displayed would normally be carried out by visibility determination unit 51 based on the assumption that the line of sight arrived at said target as determined by visual recognition determination unit 40, meaning that where two targets are displayed, it would not be possible to determine that both targets were visible unless the line of sight were made to arrive at both targets.

To address this, by using the result of determination at multiple target testing unit 60, it is possible to simultaneously carry out visibility determination with respect to the two targets even when the line of sight has not been made to arrive at the targets, making it possible to dramatically reduce the time required to carry out visibility determination.

Furthermore, based on the first determination and the second determination, visibility determination unit 51 carries out determination as to whether or not both of the two targets displayed at locations other than the location of the line of sight are nonvisible. When the result of determination at multiple target testing unit 60 is response (A, 4) or response (B, 4), visibility determination unit 51 determines that both of the two targets were nonvisible at the time that the targets were displayed.

This indicates that at the time of termination of the second response the line of sight of the user had proceeded to a location that was neither the initial position nor either of the targets nor a location midway between the two targets and had stopped there, it being thought that this was due to the fact that the line of sight had been moved to and made to stop at that other location due to the fact that neither of the two targets had been seen by the user. In such a situation as well, visibility determination unit 51 makes it possible to dramatically reduce the time required to carry out visibility determination.

By making use of the visibility determination carried out by visibility determination unit 51, which thus causes simultaneous display of two targets and reduces determination time, visual field testing unit 55 makes it possible to achieve dramatic reduction in the time required to carry out visual field testing.

Testing of mild cognitive impairment which is carried out by MCI testing unit 62 will be described next. During MCI testing, the user is instructed to look at target(s) displayed at display 13. MCI testing unit 62 is such that, for each determination result combination at multiple target testing unit 60, points (5 points being a perfect score) are assigned as described below, testing as to whether or not mild cognitive impairment exists being carried out based on the number of points. Note, however, that during testing by MCI testing unit 62, determination with respect to staring at the first response by multiple target testing unit 60 is such that the determination with respect to staring is carried out based on whether or not the line of sight stops not for 250 ms but for three times this, i.e., 750 ms.

A, 1, a: 1
A, 1, b: 1
A, 2, a: 1
A, 2, b: 5
A, 3, a: 3
A, 3, b: 5
A, 4, a: 1
A, 4, b: 3

B, 1, a: 0
B, 1, b: 0
B, 2, a: 0
B, 2, b: 5
B, 4, a: 0
B, 4, b: 3

Because it is seen in academia that there is increase in initial movement time in senile patients, because an "A" at the first response means that initial movement was delayed, points are added for all such combinations. A large number of points are assigned for response (A, 3, b), as there is a strong suspicion that MCI exists, inasmuch as the instruction to search appears not to have been understood. A large number of points are assigned for response (A, 2, b) and response (B, 2, b), inasmuch as neither target was seen despite the fact that both of the two targets were visible.

Furthermore, an intermediate number of points are assigned for response (A, 3, a), because the response was too slow. For response (A, 4, b) and response (B, 4, b), while searching for targets was not performed, considering that this may have been due to the influence of fatigue, an intermediate number of points are assigned.

MCI testing unit 62 is such that testing by multiple target testing unit 60 is carried out multiple times, a determination being made that there is a suspicion **that** MCI exists if the cumulative number of points is greater than or equal to 0.8 × the number of times that testing was carried out. For example, if testing by multiple target testing unit 60 is carried out 20 times, and the total number of points is greater than or equal to 16 points, MCI testing unit 62 would determine that there is a suspicion that MCI exists.

Second mild cognitive impairment testing at MCI testing unit 62 will be described next. During this testing, two targets of differing color are displayed at display 13, and the user is instructed to look at that of one color. For example, two targets might be respectively displayed in red and blue, and the user might be instructed to look at the target that is red.

MCI testing unit 62 is such that, during second mild cognitive impairment testing as well, for each determination result combination at multiple target testing unit 60, points (5 points being a perfect score) are assigned as described below, testing as to whether or not mild cognitive impairment exists being carried out based on the number of points.

A, 1, a: 1 if the red target is ultimately looked at; 5 if the blue target is ultimately looked at
A, 1, b: 1 if the red target is ultimately looked at; 5 if the blue target is ultimately looked at
A, 2, a: 1 if the red target is ultimately looked at; 5 if the blue target is ultimately looked at
A, 2, b: 5
A, 3, a: 3 if the red target is ultimately looked at; 5 if the blue target is ultimately looked at
A, 3, b: 5
A, 4, a: 1 if the red target is ultimately looked at; 5 if the blue target is ultimately looked at
A, 4, b: 3

B, 1, a: 0 if the red target is ultimately looked at; 5 if the blue target is ultimately looked at
B, 1, b: 0 if the red target is ultimately looked at; 5 if the blue target is ultimately looked at
B, 2, a: 0 if the red target is ultimately looked at; 5 if the blue target is ultimately looked at
B, 2, b: 5
B, 4, a: 0 if the red target is ultimately looked at; 5 if the blue target is ultimately looked at
B, 4, b: 3

MCI testing unit 62 is such that testing by multiple target testing unit 60 is carried out multiple times, a determination being made that there is a suspicion that MCI exists if the cumulative number of points is greater than or equal to 0.8 × the number of times that testing was carried out. While second mild cognitive impairment testing also provides similar action and effect as the foregoing mild cognitive impairment testing, because a large number of points are assigned for the respective determination results, it makes it possible to carry out testing in a shorter period of time.

Concentration testing carried out by concentration testing unit 65 will be described next. During concentration testing, the user is instructed to, as quickly as possible, look at target(s) displayed at display 13. Concentration testing unit 65 is such that, for each determination result combination at multiple target testing unit 60, points (5 points being a perfect score) are assigned as described below, testing as to whether or not the user is able to concentrate being carried out based on the number of points.

A, 1, a: 3
A, 1, b: 3
A, 2, a: 3
A, 2, b: 0
A, 3, a: 1
A, 3, b: 0
A, 4, a: 3
A, 4, b: 0

B, 1, a: 5
B, 1, b: 5
B, 2, a: 5
B, 2, b: 0
B, 4, a: 5
B, 4, b: 0

Concentration testing unit 65 is such that testing by multiple target testing unit 60 is carried out multiple times, a determination being made that concentration was possible if the cumulative number of points is greater than or equal to 80% of a perfect score. For example, if multiple target display response testing is carried out 20 times, and the cumulative number of points is greater than or equal to 80 points, a determination would be made that concentration was possible.

Psychogenic visual impairment determination carried out by psychogenic visual impairment testing unit 67 will be described next. During psychogenic visual impairment determination, the user is instructed to look at both of two targets in sets of targets that are sequentially displayed at display 13.

FIG. 9 shows locations at which two targets might be displayed during psychogenic visual impairment testing, in which multiple target testing unit 60, unlike the situation shown in the aforementioned FIG. 8, causes two targets to be simultaneously displayed at locations of differing distances from the location of the current line of sight.

More specifically, target display unit 33 is controlled so as to cause one of the two targets (the inner target) to be displayed at a location at which the vertical angle and horizontal angle are both within 10° from a location at front and center which is the location of the current line of sight, and so as to cause the other target (the outer target) to be displayed at a location at which the vertical angle and horizontal angle are both not less than 20°. Furthermore, the two targets are displayed at locations such as will cause the angle formed by lines respectively connecting the targets with the location of the current target at front and center to be 90°.

Psychogenic visual impairment is a condition frequently found among children, in which visual acuity is reduced as a result of psychological stress. In accordance with the present embodiment, it is assumed for a patient who has psychogenic visual impairment that as testing proceeds the visual field of the user will grow narrower and there will be increase in the frequency with which only the inner target is visually recognized.

In accordance with the present embodiment, first determination and second determination are carried out multiple times by multiple target testing unit 60; and with respect to the second response, the number of times that the determination was "1" and the inner target was seen, the number of times that the determination was "1" and the outer target was seen, and the number of times that the determination was "2" are counted, and the fractional number of times that the inner target was seen is calculated.

Psychogenic visual impairment testing unit 67 is such that 100 iterations of testing are carried out by multiple target testing unit 60, the determination being that there is a suspicion that psychogenic visual impairment exists if the fractional number of times that the inner target was seen during the final 50 iterations is greater than or equal to 1.5 times the fractional number of times that the inner target was seen during the initial 50 iterations.

Above, description has been given with respect to characteristic testing system 1 associated with the present embodiment; in accordance with the present embodiment, by causing testing with respect to movement of the line of sight of a user at a time when two targets are displayed to be carried out by multiple target testing unit 60, it is possible for determination with respect to various characteristics of the user to be efficiently carried out by characteristic testing unit 50.

Moreover, the present invention is not limited to the foregoing embodiment but admits of any number of variations without departing from the gist of the present invention. For example, whereas a polar coordinate system was employed for lines of sight and line of sight position information, it is also possible to employ a three-dimensional rectangular coordinate system therefor.

Furthermore, whereas in the foregoing embodiment independent displays were used for the display for the left eye and the display for the right eye, it is also possible to use a single large display that has been divided into a region for the right eye and a region for the left eye.

Furthermore, whereas in the foregoing embodiment, testing with respect to movement of the line of sight of a user by the multiple target testing unit was carried out at a time when a plurality of targets in the form of two targets were simultaneously displayed, it is of course also possible to carry out testing with respect to movement of the line of sight of the user at a time when three or more targets are simultaneously displayed.

Where determination is carried out by the visibility determination unit at a time when three or more targets are simultaneously displayed, it will be possible to determine that all targets are visible when the line of sight of the user moves to and stops at a location midway thereamong which corresponds to a region at the center of the plurality of targets. Furthermore, it will likewise be possible to determine that all targets are nonvisible when the line of sight of the user proceeds to and stops at a location other than the initial position, any of the targets, or midway among the plurality of targets.

### EXPLANATION OF REFERENCE NUMERALS

- 1: Characteristic testing system
- 10: HMD
- 11: Case
- 13: Display
- 14: Convex lens
- 15: Camera
- 16: Hot mirror
- 18: Emitter
- 30: Control apparatus
- 31: Line of sight detection unit
- 33: Target display unit
- 40: Visual recognition determination unit
- 41: Collision determination unit
- 50: Characteristic testing unit
- 51: Visibility determination unit
- 52: Visibility determination region establishment unit
- 55: Visual field testing unit
- 60: Multiple target testing unit
- 62: MCI (mild cognitive impairment) testing unit
- 65: Concentration testing unit
- 67: Psychogenic visual impairment testing unit
- 70: Arithmetic unit
- 75: Storage device
- 76: Line of sight log storage unit
- 77: Target storage unit
- 80: Cable
- 91: Initial position of line of sight
- 92: Target
- A: Line segment (shortest path)
- C: Arrival path
- X: Visibility determination region

## Claims

1. A characteristic testing system (1) **characterized in that** it comprises:
a display (13);
a line of sight detection unit (31) configured to detect a line of sight of a user and to output line of sight information pertaining to a direction of the line of sight;
a target display unit (33) configured to cause targets (92) to be displayed at the display; and
a characteristic testing unit (50) configured to control the target display unit,
**characterized in that**,
the characteristic testing unit comprises:
a multiple target testing unit (60) configured to display a plurality of the targets simultaneously at the display, and to determine (i) whether or not, immediately following display of targets, the line of sight remained stopped at its initial position, and then (ii) whether or not the line of sight proceeds directly to and stops at a location midway among the plurality of the targets; and
a visibility determination unit (51) configured to, when the targets have been displayed at locations not in the line of sight, determine whether or not the targets were visible to the user, wherein
the visibility determination unit is configured such that, when the plurality of the targets are simultaneously displayed at the display by the multiple target testing unit, it is determined that all of the plurality of the targets are visible if the line of sight proceeds to and stops at a location midway among the plurality of the targets.

2. The characteristic testing system according to claim 1 **characterized in that**
the multiple target testing unit (60) is further configured to determine whether or not the line of sight stops at a location other than the plurality of the targets, midway among the plurality of the targets, or its initial position, and
the visibility determination unit is configured such that, when the plurality of the targets are simultaneously displayed at the display by the multiple target testing unit, it is determined that all of the plurality of the targets are nonvisible if the line of sight proceeds to and stops at a location other than the initial position, any of the targets, or midway among the plurality of the targets.

3. The characteristic testing system according to any one of claims 1 or 2 **characterized in that** the multiple target testing unit (60) is configured to cause two of the targets to be simultaneously displayed at locations equidistant from a location of the line of sight.

4. The characteristic testing system according to any one of claims 1 through 3 **characterized in that** the target display unit causes the plurality of the targets to be displayed in different colors.

## Patentansprüche

1. Eigenschaftsprüfsystem (1), **dadurch gekennzeichnet, dass** es umfasst:
eine Anzeige (13);
eine Sichtlinienerfassungseinheit (31), die dazu eingerichtet ist, eine Sichtlinie eines Benutzers zu erfassen und Sichtlinieninformationen bezüglich einer Richtung der Sichtlinie auszugeben;
eine Zielmarkenanzeigeeinheit (33), die dazu eingerichtet ist, Zielmarken (92) auf der Anzeige anzeigen zu lassen; und
eine Eigenschaftsprüfeinheit (50), die dazu eingerichtet ist, die Zielmarkenanzeigeeinheit zu steuern,
**dadurch gekennzeichnet, dass**
die Eigenschaftsprüfeinheit umfasst:
eine Mehrfachzielmarken-Prüfeinheit (60), die dazu eingerichtet ist, eine Vielzahl der Zielmarken gleichzeitig auf der Anzeige anzuzeigen und zu bestimmen, (i) ob die Sichtlinie unmittelbar nach dem Anzeigen der Zielmarken an ihrer Ausgangsposition stehen geblieben ist oder nicht, und dann (ii) ob sich die Sichtlinie direkt zu einer Stelle in der Mitte zwischen der Vielzahl der Zielmarken bewegt und dort anhält oder nicht; und
eine Sichtbarkeitsbestimmungseinheit (51), die dazu eingerichtet ist, wenn die Zielmarken an Stellen außerhalb der Sichtlinie angezeigt worden sind, zu bestimmen, ob die Zielmarken für den Benutzer sichtbar waren oder nicht, wobei die Sichtbarkeitsbestimmungseinheit derart eingerichtet ist, dass, wenn die Vielzahl der Zielmarken durch die Mehrfachzielmarken-Prüfeinheit gleichzeitig auf der Anzeige angezeigt wird, bestimmt wird, dass alle der Vielzahl der Zielmarken sichtbar sind, wenn sich die Sichtlinie zu einer Stelle in der Mitte zwischen der Vielzahl der Zielmarken bewegt und dort anhält.

2. Eigenschaftsprüfsystem nach Anspruch 1, **dadurch gekennzeichnet, dass**
die Mehrfachzielmarken-Prüfeinheit (60) ferner dazu eingerichtet ist, zu bestimmen, ob die Sichtlinie an einer Stelle anhält oder nicht, die weder die Vielzahl der Zielmarken noch die Mitte zwischen der Vielzahl der Zielmarken noch ihre Ausgangsposition ist, und
die Sichtbarkeitsbestimmungseinheit derart eingerichtet ist, dass, wenn die Vielzahl der Zielmarken durch die Mehrfachzielmarken-Prüfeinheit gleichzeitig auf der Anzeige angezeigt wird, bestimmt wird, dass alle der Vielzahl der Zielmarken nicht sichtbar sind, wenn sich die Sichtlinie zu einer Stelle bewegt und dort anhält, die weder die Ausgangsposition noch eine der Zielmarken noch die Mitte zwischen der Vielzahl der Zielmarken ist.

3. Eigenschaftsprüfsystem nach einem der Ansprüche 1 oder 2, **dadurch gekennzeichnet, dass** die Mehrfachzielmarken-Prüfeinheit (60) dazu eingerichtet ist, zwei der Zielmarken gleichzeitig an Stellen anzeigen zu lassen, die von einer Stelle der Sichtlinie gleich weit entfernt sind.

4. Eigenschaftsprüfsystem nach einem der Ansprüche 1 bis 3, **dadurch gekennzeichnet, dass** die Zielmarkenanzeigeeinheit die Vielzahl der Zielmarken in unterschiedlichen Farben anzeigen lässt.

## Revendications

1. Système de test de caractéristiques (1), **caractérisé en ce qu'**il comprend :
un afficheur (13) ;
une unité de détection de ligne de visée (31) configurée pour détecter une ligne de visée d'un utilisateur et pour délivrer des informations de ligne de visée relatives à une direction de la ligne de visée ;
une unité d'affichage de cibles (33) configurée pour faire afficher des cibles (92) sur l'afficheur ; et
une unité de test de caractéristiques (50) configurée pour commander l'unité d'affichage de cibles,
**caractérisé en ce que**
l'unité de test de caractéristiques comprend :
une unité de test à cibles multiples (60) configurée pour afficher une pluralité des cibles simultanément sur l'afficheur, et pour déterminer (i) si, immédiatement après l'affichage des cibles, la ligne de visée est restée arrêtée ou non à sa position initiale, puis (ii) si la ligne de visée se dirige directement vers un emplacement à mi-chemin entre la pluralité des cibles et s'y arrête ou non ; et
une unité de détermination de visibilité (51) configurée pour, lorsque les cibles ont été affichées à des emplacements qui ne se trouvent pas dans la ligne de visée, déterminer si les cibles étaient visibles ou non pour l'utilisateur,
dans lequel l'unité de détermination de visibilité est configurée de telle sorte que, lorsque la pluralité des cibles est affichée simultanément sur l'afficheur par l'unité de test à cibles multiples, il est déterminé que toutes les cibles de la pluralité de cibles sont visibles si la ligne de visée se dirige vers un emplacement à mi-chemin entre la pluralité des cibles et s'y arrête.

2. Système de test de caractéristiques selon la revendication 1, **caractérisé en ce que**
l'unité de test à cibles multiples (60) est en outre configurée pour déterminer si la ligne de visée s'arrête ou non à un emplacement autre que la pluralité des cibles, un emplacement à mi-chemin entre la pluralité des cibles ou sa position initiale, et
l'unité de détermination de visibilité est configurée de telle sorte que, lorsque la pluralité des cibles est affichée simultanément sur l'afficheur par l'unité de test à cibles multiples, il est déterminé que toutes les cibles de la pluralité de cibles sont non visibles si la ligne de visée se dirige vers un emplacement autre que la position initiale, l'une quelconque des cibles ou un emplacement à mi-chemin entre la pluralité des cibles, et s'y arrête.

3. Système de test de caractéristiques selon l'une quelconque des revendications 1 ou 2, **caractérisé en ce que** l'unité de test à cibles multiples (60) est configurée pour faire afficher deux des cibles simultanément à des emplacements équidistants d'un emplacement de la ligne de visée.

4. Système de test de caractéristiques selon l'une quelconque des revendications 1 à 3, **caractérisé en ce que** l'unité d'affichage de cibles fait afficher la pluralité des cibles dans des couleurs différentes.
